# EUROPEAN PATENT APPLICATION

(11) **EP 4 806 471 A1**
(43) Date of publication of application: **16.09.2026**
(21) Application number: 25752341.5
(22) Date of filing: 05.02.2025
(51) Int. Cl.: A61L 27/14, A61L 27/52, A61L 27/58, A61L 27/60, A61L 27/16, A61L 27/18, A61L 27/20, A61L 27/22

(54) **COMPOSITION FOR FORMING ADIPOSE TISSUE-LIKE STRUCTURE AND METHOD FOR PRODUCING ADIPOSE TISSUE-LIKE STRUCTURE**

(30) Priority: 07.02.2024 KR 20240018658
(71) Applicant: Aldaver Inc., Daejeon 34051 (KR)
(72) Inventor: KIM, Jin-Oh, Daejeon 34040 (KR); LEE, Ye Rim, Daejeon 35202 (KR); OH, Jaeho, Daejeon 34051 (KR); SON, Ryeo Jin, Daejeon 34052 (KR)
(74) Representative: dompatent
(86) International application number: PCT/KR2025/001656
(87) International publication number: WO 2025/170295

(57) **Abstract**

The present invention relates to a composition for forming an adipose tissue-like structure comprising a biocompatible polymer in a dispersed form in the composition, a biocompatible hydrogel contained together in the form of a plurality of spherical particles, an acrylamide-based monomer, an initiator, a crosslinking agent, and a solvent, and a method for producing an adipose tissue-like structure using the same.

## Description

### [Technical Field]

The present application claims the benefit of priority based on Korean Patent Application No. 10-2024-0018658 filed on February 07, 2024, the entire contents of which are incorporated herein as part of this specification.

The present invention relates to a composition for forming an adipose tissue-like structure and a method for producing the adipose tissue-like structure.

### [Background Art]

Adipose tissue, composed of fat cells, acts as an endocrine organ that interacts closely with other organs and plays a crucial role in regulating metabolism. In the fields of modern medicine and regenerative medicine, damage to and deficiency of these adipose tissues cause many health and aesthetic problems. Obesity, metabolic syndrome, and age-related reduction in adipose tissue resulting from excessive subcutaneous or visceral fat are emerging as serious medical problems in our society, leading to increased risk of diseases such as cardiovascular disease, diabetes, and arthritis, as well as a decline in the quality of life of patients.

To address these issues, research on adipose tissue regeneration and the formation of similar structures has been actively conducted in the fields of tissue engineering and stem cell technology in recent years, and innovative approaches to adipose tissue are being investigated in various fields, such as cell regeneration, biomaterial engineering, and stem cell transplantation technology.

However, it is difficult to effectively resolve damage and deficiency of adipose tissue with current treatment and regenerative technologies, and fat grafting, fat injections, or cosmetic surgery are merely temporary solutions, with limitations in their long-term sustainability and stability.

In this regard, structures with physical properties similar to human adipose tissue have recently emerged as a solution. Adipose tissue-like structures can be potentially utilized in various application fields. Such structures can contribute as innovative solutions for obesity management, cosmetic surgery, fat grafting, new drug development and testing, and body part restoration.

However, while such adipose tissue-like structures can exhibit appearances and functions similar to actual human tissue, they are not perfectly identical because human tissue consists of various tissue layers with distinct physical properties. Therefore, there is a limitation in that each tissue layer must be implemented individually to simulate realistic surgery for surgery on the human body.

In particular, during human surgery, adipose tissue is frequently used to pull and suture relatively hard skin layers (e.g., dermis or muscle layer), and during surgery, the process of cutting or removing adipose tissue is also frequently performed in the process of locating target organs; therefore, it is urgent to present a solution that can implement physical properties similar to actual human adipose tissue.

### [Prior Art Document]

(Patent Document 1) Korean Laid-open Patent Publication No. 10-2019-0080830

### [Disclosure]

### [Technical Problem]

It is an object of the present invention to provide a composition for forming an adipose tissue-like structure implemented to enable simulation similar to an actual human surgical process.

It is another object of the present invention to provide a method for producing an adipose tissue-like structure.

### [Technical Solution]

One embodiment of the present invention provides a composition for forming an adipose tissue-like structure comprising a biocompatible polymer in a dispersed form in the composition, a plurality of spherical particle-shaped biocompatible hydrogels, an acrylamide-based monomer, an initiator, a crosslinking agent, and a solvent.

The biocompatible polymer and biocompatible hydrogel may each comprise one or more of gelatin, gelatin methacrylate, collagen, alginate, fibrinogen, fibrin, hyaluronic acid, methyl cellulose, chitosan, chitin, Extracel^{™}, pectin, polylactic acid (PLA), polyacrylic acid (PAA), polyphosphoric acid, and synthetic peptide.

The acrylamide-based monomer may comprise one or more of acrylamide, N-isopropylacrylamide(NIPAM), N-tert-butyl acrylamide, N,N-diethylacrylamide, N-ethylacrylamide, N-n-propylacrylamide, N,N-ethylmethylacrylamide, N-isopropylmethacrylamide, N-hydroxyethylacrylamide, N-(isobutoxymethyl)acrylamide, N-tert-butyl methacrylamide, N,N-diethylmethacrylamide, and N-ethylmethacrylamide.

The plurality of spherical particle-shaped biocompatible hydrogels may contain ionic functional groups.

The ionic functional group may be one or more selected from the group consisting of a carboxyl group, a hydroxyl group, a thiol group, an amine group, a sulfonate group, an ammonium group, a urea group, a thiourea group, an imidazole group, a phosphate group, a sulfonic acid group, and a phosphonic acid group.

The biocompatible polymer in a dispersed form in the composition may be contained in an amount of 0.2 to 1 parts by weight based on the total 100 parts by weight of the composition.

The plurality of spherical particle-shaped biocompatible hydrogels may be contained in an amount of 40 to 60 parts by weight based on the total 100 parts by weight of the composition.

The acrylamide-based monomer may be contained in an amount of 3 to 16 parts by weight based on the total 100 parts by weight of the composition.

The multiple spherical particles may be in the form of granules.

In the plurality of spherical particle-shaped biocompatible hydrogels, the average particle size of each individual particle may be 0.1 to 3 mm.

The composition may be a composition in which the plurality of spherical particle-shaped biocompatible hydrogels are soaked in a solution containing the biocompatible polymer in a dispersed form in the composition, the acrylamide-based monomer, the initiator, and the crosslinking agent in the solvent.

Another embodiment of the present invention provides a method for producing an adipose tissue-like structure comprising the steps of: (1) preparing a plurality of spherical particle-shaped biocompatible hydrogels by dropping a biocompatible hydrogel into a solution containing a polyvalent metal ion compound; (2) preparing a mixed solution by adding the plurality of spherical particle-shaped biocompatible hydrogels prepared in the step (1) into a network solution containing an acrylamide-based monomer, an initiator, a crosslinking agent, a biocompatible polymer, and a solvent; and (3) curing the mixed solution of the step (2).

The mixing ratio of the plurality of spherical particle-shaped biocompatible hydrogels of the step (1) and the network solution of the step (2) may be a weight ratio of 1:0.7 to 1:1.3.

After the step (1), the method may further comprise the step of soaking the plurality of spherical particle-shaped biocompatible hydrogels in a network precursor solution containing an acrylamide-based monomer, an initiator, and a crosslinking agent.

The polyvalent metal ion compound may comprise one or more of sodium chloride, zirconium chloride, iron chloride, calcium chloride, magnesium chloride, aluminum chloride, aluminum sulfate, potassium alum, iron chloride alum, ammonium alum, ferric sulfate, aluminum hydroxide, aluminum silicate, aluminum phosphate, ferric citrate, magnesium oxide, calcium phosphate, calcium carbonate, calcium oxide, zinc oxide, and zinc sulfate.

### [Advantageous Effects]

According to the present invention, the composition capable of forming the adipose tissue-like structure comprises the acrylamide-based monomer, the initiator, and the crosslinking agent, along with the biocompatible polymer in a dispersed form in the composition and the plurality of spherical particle-shaped biocompatible hydrogels, and thus as the plurality of spherical particle-shaped biocompatible hydrogels have a granular form, the adipose tissue-like structure produced by curing the composition not only exhibits a shape similar to that of fat cells found in actual human organs, but also has the advantage of being soft in physical properties, having a non-sticky surface, and securing physical and mechanical properties sufficient to enable actual suturing during surgical simulation.

### [Description of Drawings]

FIG. 1 is a schematic diagram showing the process of producing an adipose tissue-like structure using the composition for forming an adipose tissue-like structure according to an example of the present invention.
FIGS. 2 to 5 show images of the adipose tissue-like structures formed through the compositions for forming an adipose tissue-like structure according to an example of the present invention and a comparative example.

### [Best Mode]

The present invention will be described in more detail below. First of all, the terms and words used in the present specification and claims should not be construed as limited to ordinary or dictionary terms, and should be construed in a sense and concept consistent with the technical idea of the present invention, based on the principle that the inventor can properly define the concept of a term to describe his invention in the best way possible. Therefore, since the configuration shown in the embodiments described in the present specification is only one of the most preferred embodiments of the present invention, and does not represent all of the technical spirit of the present invention, it should be understood that various equivalents and modifications may be substituted for them at the time of filing the present application.

Throughout the specification of the present application, when a part is described as "comprise" a certain component, this means that, unless specifically stated otherwise, it does not exclude other components but may further comprise other components.

In addition, descriptions that specify components by limiting or adding them may be applied to all inventions unless there are special limitations, and are not limited to specific inventions.

In addition, throughout the description and the claims of the present application, singular notations comprise plural notations unless otherwise noted.

In addition, throughout the description and the claims of the present application, the term "or" comprises "and" unless otherwise noted. Therefore, "comprising A or B" means all three cases of comprising A, comprising B, or comprising both A and B.

In addition, all numerical ranges comprise the values at both ends and all intermediate values in between, unless explicitly stated to be excluded.

Throughout the specification of the present application, the average particle size of the particles may be, for example, the median diameter (D50) measured using a laser particle size distribution meter.

### Composition for forming adipose tissue-like structure

The present invention relates to a composition for forming an adipose tissue-like structure which comprises an acrylamide-based monomer, an initiator, a crosslinking agent, and a solvent, along with a biocompatible polymer and a biocompatible hydrogel, wherein the biocompatible hydrogel within the adipose tissue-like structure produced by curing has the form of granules containing multiple particles, and thus the adipose tissue-like structure exhibits a shape similar to that of fat cells found in actual human organs which contains spherical fat cells embedded in loose connective tissue, is soft in physical properties, has a non-sticky surface, and secures physical and mechanical properties sufficient to enable actual suturing during surgical simulation.

Hereinafter, each component of the composition for forming the adipose tissue-like structure according to one embodiment of the present invention will be described.

### (1) Biocompatible hydrogel and biocompatible polymer

The biocompatible hydrogel is a substance capable of exhibiting a form similar to fat cells found in human organs, and may be contained in the form of a plurality of spherical particles within the composition for forming the adipose tissue-like structure according to one embodiment of the present invention.

The biocompatible hydrogel may refer to a structure in which a biocompatible polymer, such as gelatin, gelatin methacrylate, collagen, alginate, fibrinogen, fibrin, hyaluronic acid, methyl cellulose, chitosan, chitin, Extracel^{™}, pectin, polylactic acid (PLA), polyacrylic acid (PAA), polyphosphoric acid, and synthetic peptide, forms a network structure in which three-dimensional crosslinks are formed by physical or chemical bonding, thereby containing an aqueous solvent such as water inside. The biocompatible hydrogel can be formed into spherical particles, and in the case of the hydrogel according to the present invention, the type of substance is not limited as long as it exhibits biocompatibility, but preferably, a hydrogel containing alginate can be used.

In the case of the plurality of spherical particle-shaped biocompatible hydrogels, the average particle size of the individual particles of the hydrogel in the form of multiple spherical particles may be 0.1 to 3 mm, for example, 0.5 to 2 mm or 0.5 to 1 mm.

If the average particle size of the individual particles of the hydrogel in the form of multiple spherical particles is within the range described above, it is possible to more realistically realize fat cells contained in the fat layer of the human body.

In one embodiment of the present invention, the plurality of spherical particle-shaped biocompatible hydrogels may be in a form soaked in the composition described above, and the biocompatible hydrogels embodied in the form of spherical particles within the entire composition may be soaked in a solution containing a biocompatible polymer in a dispersed form in the composition, an acrylamide-based monomer, an initiator, and a crosslinking agent in a solvent.

In particular, the biocompatible hydrogel contained in the form of a plurality of spherical particles may be in the form of granules formed by aggregating a plurality of spherical particles, and according to this structural characteristic, in the adipose tissue-like structure formed through the composition for forming the adipose tissue-like structure according to one embodiment of the present invention, the biocompatible hydrogel in the form of granules can exhibit properties that are morphologically and physically similar to fat cells in the actual human body.

Also, in one embodiment of the present invention, the biocompatible hydrogel contained in the form of the plurality of spherical particles may contain an ionic functional group.

Since the biocompatible hydrogel contained in the composition in the form of a plurality of spherical particles contains an ionic functional group, when the biocompatible hydrogel containing the ionic functional group is dropped into a solution containing a polyvalent metal ion compound according to the method for producing an adipose tissue-like structure to be described below, the ionic functional group contained in the biocompatible hydrogel is crosslinked by polyvalent metal ions generated from the polyvalent metal compound in the solution, thereby forming the biocompatible hydrogel in the form of spherical particles.

The ionic functional group contained in the biocompatible hydrogel described above may not be limited to any specific type as long as it is capable of forming a spherical particle-shaped biocompatible hydrogel by crosslinking the biocompatible hydrogel through crosslinking with polyvalent metal ions, and may comprise, for example, one or more selected from the group consisting of a carboxyl group, a hydroxyl group, a thiol group, an amine group, a sulfonate group, an ammonium group, a urea group, a thiourea group, an imidazole group, a phosphate group, a sulfonic acid group, and a phosphonic acid group.

In one embodiment of the present invention, based on the total 100 parts by weight of the composition, the plurality of spherical particle-shaped biocompatible hydrogels may be contained in an amount of 40 to 60 parts by weight, and may be contained, for example, in an amount of 40 parts by weight or more, 41 parts by weight or more, 42 parts by weight or more, 43 parts by weight or more, 44 parts by weight or more, 45 parts by weight or more, 46 parts by weight or more, 47 parts by weight or more, 48 parts by weight or more, 49 parts by weight or more, or 50 parts by weight or more, and in an amount of 60 parts by weight or less, 59 parts by weight or less, 58 parts by weight or less, 57 parts by weight or less, 56 parts by weight or less, 55 parts by weight or less, 54 parts by weight or less, 53 parts by weight or less, 52 parts by weight or less, or 51 parts by weight or less.

If the content of the plurality of spherical particle-shaped biocompatible hydrogels is within the range described above, the adipose tissue-like structure formed through the composition according to the present invention may exhibit physical properties particularly similar to fat cells within the fat layer of the human body. If the content of the plurality of spherical particle-shaped biocompatible hydrogels exceeds 60 parts by weight, based on the total 100 parts by weight of the composition, since the curing process for forming an adipose tissue-like structure may not proceed well. Therefore, the content of the biocompatible hydrogel is appropriately controlled within the range described above.

In one embodiment of the present invention, the types of the biocompatible polymer in a dispersed form in the composition may comprise, for example, gelatin, gelatin methacrylate, collagen, alginate, fibrinogen, fibrin, hyaluronic acid, methyl cellulose, chitosan, chitin, Extracel^{™}, pectin, polylactic acid (PLA), polyacrylic acid (PAA), polyphosphoric acid, synthetic peptides, etc. which are comprised in the biocompatible hydrogel.

In one embodiment of the present invention, the biocompatible polymer in a dispersed form in the composition may be contained in an amount of 0.2 to 1 parts by weight based on the total 100 parts by weight of the composition, and may be contained, for example, in an amount of 0.3 to 0.9 parts by weight, 0.4 to 0.8 parts by weight, and preferably in an amount of 0.35 to 0.75 parts by weight.

If the content of the biocompatible polymer in a dispersed form in the composition is less than 0.2 parts by weight, the mechanical properties of the adipose tissue-like structure formed through the composition are lower than those of the fat layer of the actual human body, and thus, when external pressure is applied, it may be easily damaged, and it may not be easily cut with a scalpel or may be easily torn, making it unsuitable for simulating a surgical procedure similar to that of the human body through the structure.

If the content of the biocompatible polymer in a dispersed form in the composition exceeds 1 part by weight, since it may exhibit physical properties that are harder than the actual human body fat layer, which may cause a foreign sensation, it is appropriately controlled within the range described above.

### (2) Acrylamide-based monomer

The composition for forming an adipose tissue-like structure according to the present invention further comprises an acrylamide-based monomer along with the biocompatible polymer and biocompatible hydrogel. The acrylamide-based monomer is a type of substance capable of forming a hydrogel, and it changes into polyacrylamide after the composition according to the present invention is cured. As the composition according to the present invention is cured, the acrylamide-based monomer forms a hydrogel network, thereby performing the role of mimicking connective tissue in the fat layer of the actual human body.

In one embodiment of the present invention, the acrylamide-based monomer may be one or more selected from the group consisting of acrylamide, N-isopropylacrylamide(NIPAM), N-tert-butyl acrylamide, N,N-diethylacrylamide, N-ethylacrylamide, N-n-propylacrylamide, N,N-ethylmethylacrylamide, N-isopropylmethacrylamide, N-hydroxyethylacrylamide, N-(isobutoxymethyl)acrylamide, N-tert-butyl methacrylamide, N,N-diethylmethacrylamide and N-ethylmethacrylamide, and for example, acrylamide can be used.

In one embodiment of the present invention, the acrylamide-based monomer may be contained in an amount of 3 to 16 parts by weight based on the total 100 parts by weight of the composition, and may be contained, for example, in an amount of 3.5 to 14 parts by weight, preferably 4 to 12 parts by weight.

If the content of the acrylamide-based monomer is less than 3 parts by weight based on the total 100 parts by weight of the composition, there may be problems in that the biocompatible hydrogel contained in the composition in the form of the plurality of particles may be weaker than the physical properties of fat cells of the actual human body, causing the adipose tissue-like structure to tear easily when external pressure is applied, or the curing speed may be reduced during the curing process of the composition. If the content of the acrylamide-based monomer exceeds 16 parts by weight, the adipose tissue-like structure formed through the composition may exhibit physical properties that are harder than the fat layer of the actual human body, which may result in a foreign sensation. Therefore, the content of the acrylamide-based monomer is appropriately controlled within the range described above so that the adipose tissue-like structure can exhibit physical properties similar to the fat layer of the human body.

### (3) Other components

The composition for forming the adipose tissue-like structure of the present invention may further comprise a crosslinking agent and an initiator in addition to the components described above for controlling physical properties.

The crosslinking agent is a substance that acts to crosslink between the network formed through the biocompatible polymer, biocompatible hydrogel, and acrylamide-based monomer in the composition. Specifically, the crosslinking agent can play a role in further improving the mechanical strength and chemical stability of the adipose tissue-like structure by crosslinking between the polyacrylamide chains formed by the curing of the acrylamide-based monomer.

Therefore, the crosslinking agent may not be limited to any specific type as long as it is a substance capable of crosslinking the network formed through the biocompatible polymer, biocompatible hydrogel, and acrylamide-based monomer of the present invention, and may be, for example, at least one selected from the group consisting of N,N'-methylenebisacrylamide (MBA), trimethylolpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triallylamine, ethylene glycol diglycidyl ether, propylene glycol, glycerin, and ethylene carbonate.

In the case of the crosslinking agent, it may be contained in an amount of 0.001 to 0.05 parts by weight based on the total 100 parts by weight of the composition according to the present invention, and may be contained, for example, 0.003 to 0.04 parts by weight, preferably 0.005 to 0.03 parts by weight. If the content of the crosslinking agent is less than the range described above, sufficient crosslinking may not be achieved to provide mechanical strength and chemical stability to the adipose tissue-like structure, and the adipose tissue-like structure formed through the composition may be weaker than the physical properties of fat cells of the actual human body, thereby causing the adipose tissue-like structure to tear easily when external pressure is applied. If the content of the crosslinking agent exceeds the range described above, there is a problem in that excessive cross-linking occurs, and thus the adipose tissue-like structure exhibits physical properties that are harder than the fat layer of the actual human body, which may result in a foreign sensation.

The initiator is a substance used to initiate a chain reaction for the curing of the composition, and specifically, refers to a substance that easily generates radicals using a light source such as UV or heat.

The initiator may not be limited to any type as long as it is a substance capable of initiating a curing reaction by a light source or by heat, and may be, for example, one or more of riboflavin (vitamin B2), lithium phenyl-(2,4,6-trimethyl benzoyl) phosphinate (LAP), benzyl dimethyl ketal, acetophenone, benzoin methylether, diethoxyacetophenone, benzoyl phosphine oxide, 1-hydroxycyclohexyl phenyl ketone, tris(bipyridine)ruthenium(II) chloride (Ru(bpy3)²⁺), ammonium persulfate (APS), horse radish peroxide (HRP) and H₂O₂ (hydrogen peroxide), persulfates such as potassium persulfate and ammonium persulfate; azo compounds such as 4,4-azobis(4-cyanovaleric acid), dimethyl-2,2'-azobis(2-methylpropionate), 2,2-azobis(2-amidinopropane) dihydrochloride, 2,2-azobis(2-methylpropionitrile), 2,2-azobis-2-methyl-N-1,1-bis(hydroxymethyl)-2-hydroxyethylpropioamide, 2,2'-azobis(2,4-dimethylvaleronitrile), 2,2'-azobisisobutyronitrile, and 1,1'-azobis(1-cyclohexanecarbonitrile); peroxides such as methyl ethyl peroxide, di-t-butyl peroxide, acetyl peroxide, dicumyl peroxide, lauroyl peroxide, benzoyl peroxide, t-butylperoxy-2-ethylhexanoate, di-isopropylperoxydicarbonate, and di-t-butylperoxyisophthalate, and preferably, ammonium persulfate (APS) may be used.

In the case of the initiator, it may be contained in an amount of 0.008 to 0.08 parts by weight based on the total 100 parts by weight of the composition according to the present invention. If the content of the initiator is less than the range described above, the curing process of the composition according to the present invention may not proceed smoothly, the curing time may be excessively delayed, or the adipose tissue-like structure formed through the composition may become weaker than the physical properties of fat cells of the actual human body, thereby causing the adipose tissue-like structure to tear easily when external pressure is applied. If the content of the initiator exceeds the range described above, there is a possibility that rapid radical reactions may occur, causing side reactions or unintended effects on other components of the composition according to the present invention, and there is a problem in that excessive residual components remain after curing, causing the surface of the adipose tissue-like structure to become sticky, and the adipose tissue-like structure to exhibit physical properties that are harder than the fat layer of the actual human body, which may result in a foreign sensation.

The composition for forming the adipose tissue-like structure according to the present invention may further comprise, in addition to the crosslinking agent and initiator, additives such as a surfactant, a plasticizer, an (thermal) polymerization inhibitor, a stabilizer, an antifoaming agent, a diluent, and a viscosity modifier. The additives may be contained in a minimum amount sufficient for the described action to occur in terms of economic aspects, and preferably, may be contained in an amount of 0.01 to 5 parts by weight based on the total 100 parts by weight of the composition.

### (4) Solvent

In one embodiment of the present invention, the solvent in the composition is not limited to any specific type as long as it can effectively dissolve each component, such as the biocompatible polymer, biocompatible hydrogel, acrylamide-based monomer, initiator, and crosslinking agent, but, for example, an aqueous solvent such as water may be used.

The content of the solvent may be contained in an amount such that each component in the composition can be contained within a preferred range according to the purpose of the present invention.

### Method for producing adipose tissue-like structure

Another embodiment of the present invention provides a method for producing an adipose tissue-like structure using the composition for forming the adipose tissue-like structure.

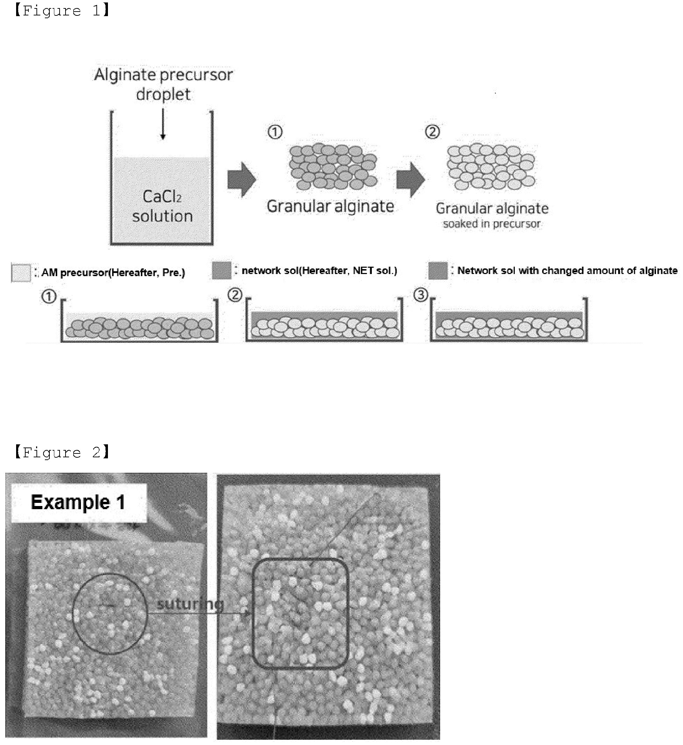

FIG. 1 shows a method for producing an adipose tissue-like structure according to one embodiment of the present invention.

Referring to FIG. 1, the method for producing the adipose tissue-like structure comprises the steps of: (1) preparing a plurality of spherical particle-shaped biocompatible hydrogels by dropping a biocompatible hydrogel solution into a solution containing a polyvalent metal ion compound; (2) preparing a mixed solution by adding the plurality of spherical particle-shaped biocompatible hydrogels prepared in the step (1) into a network solution containing an acrylamide-based monomer, an initiator, a crosslinking agent, a biocompatible polymer, and a solvent; and (3) curing the mixed solution of the step (2).

Hereinafter, the method for producing the adipose tissue-like structure is described in detail step by step below.

First, (1) a plurality of spherical particle-shaped biocompatible hydrogels are prepared by dropping a biocompatible hydrogel solution into a solution containing a polyvalent metal ion compound.

As the biocompatible hydrogel is the same as described in the composition for forming the adipose tissue-like structure, a detailed description thereof is omitted.

The biocompatible hydrogel solution can be formed by dissolving a biocompatible polymer capable of forming a hydrogel, such as gelatin, gelatin methacrylate, collagen, alginate, fibrinogen, fibrin, hyaluronic acid, methyl cellulose, chitosan, chitin, Extracel^{™}, pectin, polylactic acid (PLA), polyacrylic acid (PAA), polyphosphoric acid, or synthetic peptide, in an aqueous solvent, such as water.

The biocompatible hydrogel solution in the step (1) may, for example, contain 0.5 parts by weight to 3 parts by weight of the biocompatible polymer relative to 100 parts by weight of the solvent, and preferably 0.7 parts by weight to 2 parts by weight of the biocompatible polymer relative to 100 parts by weight of the solvent.

In the biocompatible hydrogel solution in the step (1), if the content of the biocompatible polymer is less than 0.5 parts by weight relative to 100 parts by weight of the solvent, the plurality of spherical particle-shaped biocompatible hydrogels formed by dropping them into a solution containing a polyvalent metal ion compound to be described later may have lower mechanical properties compared to the fat layer of the actual human body, and may be easily damaged when external pressure is applied, and may not be easily cut with a scalpel or may be easily torn, making it unsuitable for simulating a surgical procedure similar to that of the human body through the structure.

In the biocompatible hydrogel solution in the step (1), if the content of the biocompatible polymer exceeds 3 parts by weight relative to 100 parts by weight of the solvent, the plurality of spherical particle-shaped biocompatible hydrogels may exhibit physical properties that are harder than the fat layer of the actual human body, which may cause a foreign sensation. Therefore, the content of the biocompatible polymer is appropriately controlled within the range described above.

Next, the biocompatible hydrogel solution is dropped into a solution containing a polyvalent metal ion compound.

When the biocompatible hydrogel solution is dropped into a solution containing a polyvalent metal ion compound, the polyvalent metal ions promote the solidification of the biocompatible hydrogel, thereby producing a plurality of spherical particle-shaped biocompatible hydrogels.

The solution containing the polyvalent metal ion compound can be formed by dissolving the polyvalent metal ion compound in an aqueous solvent such as water.

The solution containing the polyvalent metal ion compound may, for example, contain 1 to 3 parts by weight of the polyvalent metal ion compound relative to 100 parts by weight of solvent, and preferably 1.8 to 2.5 parts by weight of the polyvalent metal ion compound relative to 100 parts by weight of solvent.

In the solution containing the polyvalent metal ion compound, if the content of the polyvalent metal ion compound is less than 1 part by weight relative to 100 parts by weight of the solvent, when the biocompatible hydrogel solution in the step (1) is dropped into a solution containing a polyvalent metal ion compound, a plurality of spherical particle-shaped biocompatible hydrogels may not be effectively formed. If the content of the polyvalent metal ion compound exceeds 3 parts by weight, the plurality of spherical particle-shaped biocompatible hydrogels finally formed may exhibit physical properties that are harder than fat cells of the actual human body, which may cause a foreign sensation. Therefore, the content of the polyvalent metal ion compound is appropriately controlled within the range described above.

The polyvalent metal ion compound may not be limited to any specific type as long as it is a compound containing a polyvalent metal ion that can promote the solidification of the biocompatible hydrogel, and it may comprise, for example, one or more of sodium chloride, zirconium chloride, iron chloride, calcium chloride, magnesium chloride, aluminum chloride, aluminum sulfate, potassium alum, iron chloride alum, ammonium alum, ferric sulfate, aluminum hydroxide, aluminum silicate, aluminum phosphate, ferric citrate, magnesium oxide, calcium phosphate, calcium carbonate, calcium oxide, zinc oxide, and zinc sulfate, and specifically calcium chloride (CaCl₂) may be used.

The hydrogel in the form of multiple spherical particles produced through the step (1) may have a granular form in which multiple individual particles agglomerate together.

After the step (1), a plurality of spherical particle-shaped biocompatible hydrogels prepared using ultrapure water, etc. may be further subjected to a washing step.

In one embodiment of the present invention, after the step (1), the method may further comprise the step of soaking the plurality of spherical particle-shaped biocompatible hydrogels in a network precursor solution containing an acrylamide-based monomer, an initiator, and a crosslinking agent.

If the step of soaking the plurality of spherical particle-shaped biocompatible hydrogels in the network precursor solution is not performed, the plurality of spherical particle-shaped biocompatible hydrogels absorb the acrylamide-based monomer, initiator, and crosslinking agent contained in the network solution to be described later, thereby reducing the overall content of the acrylamide-based monomer, initiator, and crosslinking agent in the network solution.

The network precursor solution can be formed by dissolving the acrylamide-based monomer, initiator, and crosslinking agent in an aqueous solvent, for example, water.

In the network precursor solution, the acrylamide-based monomer may be contained in an amount of 20 parts by weight to 35 parts by weight based on 100 parts by weight of solvent in the network precursor solution, and preferably may be contained in an amount of 25 parts by weight to 30 parts by weight.

If the content of the acrylamide-based monomer contained in the network precursor solution is less than 20 parts by weight based on 100 parts by weight of the solvent contained in the network precursor solution, the curing speed of the network solution is significantly reduced, and the physical properties of the adipose tissue-like structure formed through the subsequent curing process may be weak, unlike the connective tissue of the fat layer of the actual human body. If the content of the acrylamide-based monomer contained in the network precursor solution exceeds 35 parts by weight based on 100 parts by weight of the solvent contained in the network precursor solution, the adipose tissue-like structure may exhibit physical properties that are harder than the fat layer of the actual human body, which may cause a foreign sensation.

In the network precursor solution, the crosslinking agent may be contained in an amount of 0.01 parts by weight to 0.06 parts by weight based on 100 parts by weight of the content of the acrylamide-based monomer in the network precursor solution, and preferably may be contained in an amount of 0.02 parts by weight to 0.05 parts by weight.

If the content of the crosslinking agent contained in the network precursor solution is less than the range described above, sufficient crosslinking may not be achieved to provide mechanical strength and chemical stability to the adipose tissue-like structure, and the adipose tissue-like structure may be weaker than the physical properties of fat cells of the actual human body, thereby causing the adipose tissue-like structure to tear easily when external pressure is applied. If the content of the crosslinking agent contained in the network precursor solution exceeds the range described above, there is a problem in that excessive cross-linking occurs, and thus the adipose tissue-like structure exhibits physical properties that are harder than the fat layer of the actual human body, which may result in a foreign sensation.

In the network precursor solution, the initiator may be contained in an amount of 0.04 parts by weight to 0.12 parts by weight based on 100 parts by weight of the content of the acrylamide-based monomer in the network precursor solution, and preferably may be contained in an amount of 0.06 parts by weight to 0.11 parts by weight.

If the content of the initiator contained in the network precursor solution is less than the range described above, the curing process may not proceed smoothly, the curing time may be excessively delayed, or the adipose tissue-like structure may become weaker than the physical properties of fat cells of the actual human body, thereby causing the adipose tissue-like structure to tear easily when external pressure is applied. If the content of the initiator exceeds the range described above, there is a possibility that rapid radical reactions may occur, causing side reactions or unintended effects on other components in the network precursor solution, and there is a problem in that excessive residual components remain after curing, causing the surface of the adipose tissue-like structure to become sticky, and the adipose tissue-like structure to exhibit physical properties that are harder than the fat layer of the actual human body, which may result in a foreign sensation.

Next, (2) a plurality of spherical particle-shaped biocompatible hydrogels prepared in the step (1) are added to a network solution containing an acrylamide-based monomer, an initiator, a crosslinking agent, a biocompatible polymer, and a solvent to prepare a mixed solution.

In the step (2), since the acrylamide-based monomer, initiator, and biocompatible polymer are the same as those described in the composition for forming the adipose tissue-like structure, a detailed description thereof is omitted.

The network solution can be implemented as a part corresponding to 'connective tissue' in the fat layers of the actual human body in an adipose tissue-like structure formed through a curing process to be described later, and can be formed by dissolving the acrylamide-based monomer, initiator, crosslinking agent, and biocompatible polymer in an aqueous solvent, such as water, and in this case, the biocompatible polymer contained in the network solution may be contained in a dispersed form in the solution.

In one embodiment of the present invention, the acrylamide-based monomer contained in the network solution may be contained in an amount of 8 parts by weight to 32 parts by weight based on 100 parts by weight of the solvent contained in the network solution, and preferably may be contained in an amount of 10 to 29 parts by weight.

If the content of the acrylamide-based monomer contained in the network solution is less than 8 parts by weight based on 100 parts by weight of the solvent contained in the network solution, the curing speed of the network solution is significantly reduced, and the physical properties of the adipose tissue-like structure formed through the subsequent curing process may be weak, unlike the connective tissue of the fat layer of the actual human body. If the content of the acrylamide-based monomer contained in the network solution exceeds 32 parts by weight based on 100 parts by weight of the solvent contained in the network solution, the adipose tissue-like structure may exhibit physical properties that are harder than the fat layer of the actual human body, which may cause a foreign sensation.

Also, in one embodiment of the present invention, the biocompatible polymer contained in the network solution (in a form dispersed in the network solution) may be contained in an amount of 0.6 parts by weight to 2.5 parts by weight based on 100 parts by weight of the solvent contained in the network solution, and preferably may be contained in an amount of 0.8 to 2.0 parts by weight.

If the content of the biocompatible polymer is less than 0.6 parts by weight based on 100 parts by weight of the solvent contained in the network solution, the physical properties of the adipose tissue-like structure formed through the curing process may be weak, unlike the connective tissue of the fat layer of the actual human body. If the content of the biocompatible polymer contained in the above network solution exceeds 2.5 parts by weight based on 100 parts by weight of the solvent contained in the network solution, the adipose tissue-like structure may exhibit physical properties that are harder than the fat layer of the actual human body, which may cause a foreign sensation.

The crosslinking agent may be contained in an amount of 0.006 to 0.05 parts by weight based on 100 parts by weight of the solvent contained in the network solution, and for example, may be contained in an amount of 0.008 to 0.04 parts by weight, preferably 0.01 to 0.04 parts by weight. If the content of the crosslinking agent is less than the range described above, sufficient crosslinking may not be achieved to provide mechanical strength and chemical stability to the adipose tissue-like structure, and the adipose tissue-like structure may be weaker than the physical properties of fat cells of the actual human body, thereby causing the adipose tissue-like structure to tear easily when external pressure is applied. If the content of the crosslinking agent exceeds the range described above, there is a problem in that excessive cross-linking occurs, and thus the adipose tissue-like structure exhibits physical properties that are harder than the fat layer of the actual human body, which may result in a foreign sensation.

The initiator may be contained in an amount of 0.01 to 0.15 parts by weight based on 100 parts by weight of solvent contained in the network solution, and for example, may be contained in an amount of 0.01 to 0.1 parts by weight, preferably 0.03 to 0.1 parts by weight. If the content of the initiator is less than the range described above, the curing process may not proceed smoothly, the curing time may be excessively delayed, or the adipose tissue-like structure may become weaker than the physical properties of fat cells of the actual human body, thereby causing the adipose tissue-like structure to tear easily when external pressure is applied. If the content of the initiator exceeds the range described above, there is a possibility that rapid radical reactions may occur, causing side reactions or unintended effects on other components in the network solution, and there is a problem in that excessive residual components remain after curing, causing the surface of the adipose tissue-like structure to become sticky, and the adipose tissue-like structure to exhibit physical properties that are harder than the fat layer of the actual human body, which may result in a foreign sensation.

The network solution may be used with a tetramethylethylenediamine (TEMED) promoter in addition to an acrylamide-based monomer, initiator, and biocompatible polymer, and specifically may be contained in an amount of 0.008 to 0.02 parts by weight based on 100 parts by weight of solvent contained in the network solution.

In one embodiment of the present invention, the mixing ratio of the plurality of spherical particle-shaped biocompatible hydrogels in the step (1) and the network solution in the step (2) may be mixed in a weight ratio of 1:0.5 to 1:1.5, specifically in a weight ratio of 1:0.6 to 1:1.4, and more specifically in a weight ratio of 1:0.7 to 1:1.3.

If the content of the plurality of spherical particle-shaped biocompatible hydrogels in the step (1) is greater than 1:1.5, the network solution may not be effectively cured, and thus the adipose tissue-like structure may not be formed well, and if the content of the biocompatible hydrogel is less than 1:0.5, it may be difficult to achieve physical properties and texture similar to fat cells in the actual human body fat layer.

Next, (3) a mixed solution from the step (2), specifically a mixed solution of the plurality of spherical particle-shaped biocompatible hydrogels from step (1) and the network solution from step (2), is cured to produce an adipose tissue-like structure.

The curing process can be carried out, for example, by applying heat to the mixed solution in an oven at a temperature of 60 to 80 °C for 10 to 180 minutes, at which time the thermosetting agent contained in the network solution may cause a radical reaction, and thus the curing process of the mixed solution may proceed.

Hereinafter, specific examples of the present invention are presented. However, the examples described below are intended only to specifically illustrate or explain the present invention and are not intended to limit the scope of the present invention. Furthermore, any details not described herein can be technically inferred sufficiently by those skilled in the art and thus are omitted.

### Example 1: Production of adipose tissue-like structure

(1) (Preparation of alginate in the form of multiple spherical particles) 0.75 g of alginate containing carboxyl groups was dissolved in 100 g of deionized water (DI) as a solvent to prepare an aqueous alginate solution as a biocompatible hydrogel solution. 1.11 g of calcium chloride (CaCl₂) was dissolved in 100 g of deionized water as a solvent to prepare an aqueous calcium chloride solution containing a polyvalent metal ion compound. The aqueous alginate solution was dropped into the aqueous calcium chloride solution to prepare spherical particle-shaped alginate. The average particle size (D₅₀) of the individual particles of the prepared multiple spherical particle-shaped alginates is 1.0 mm, and the multiple particle-shaped alginates were gathered to form granules. The particulate alginates in a granular form are washed using deionized water.
(2) (soaking in network precursor solution) 28.2 g of acrylamide, 0.034 g of N,N'-methylenebisacrylamide (MBA) as a crosslinking agent, and 0.095 g of ammonium persulfate (APS) as a thermosetting agent were added to 100 g of deionized water as a solvent and dissolved to prepare a network precursor solution. The particulate alginate in a granular form prepared in (1) above was added to the prepared precursor solution and soaked for 180 minutes.
(3) (Mixing and curing of network solution) 28.2g of acrylamide, 0.034g of N,N'-methylenebisacrylamide (MEA) as a crosslinking agent, 0.095g of ammonium persulfate (APS) as a thermosetting agent, 1.28g of alginate as a biocompatible polymer, and 18.6 µl of tetramethylethylenediamine (TEMED) were added to 100g of deionized water as a solvent and dissolved to prepare a network solution. The particulate alginate in a granular form soaked in (2) above was taken and 130 g of the particulate alginate in a granular form was added to the network solution, and then, heat was applied in an oven at 60 °C for 60 minutes to cure the solution and thus produce an adipose tissue-like structure. The results are shown in FIG. 2.

### Examples 2 to 7: Production of adipose tissue-like structure

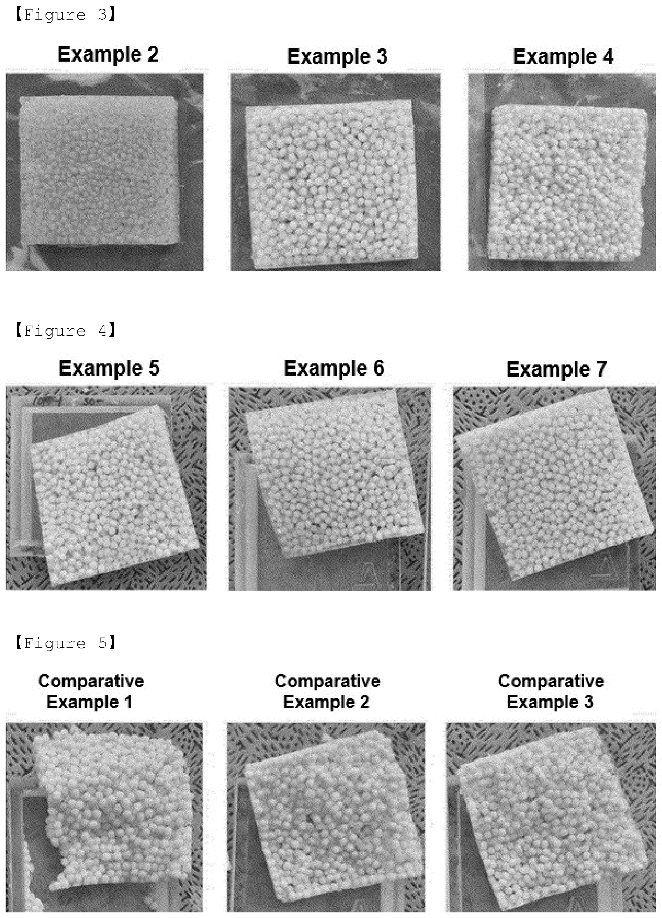

An adipose tissue-like structure was produced in the same manner as in the Example 1, except that in the Example 1, the content of the alginate and calcium chloride in step (1), the content of the acrylamide, crosslinking agent, and thermosetting agent in step (2), and the content of the acrylamide, alginate, crosslinking agent, and thermosetting agent in step (3) are as shown in Table 1 below. The results are shown in Figures 3 and 4, respectively.

**[Table 1]**

| (Unit: g) | Preparation of particulate alginate | | | | Soaking in network precursor solution | | | | Network solution | | | | |
|---|---|---|---|---|---|---|---|---|---|---|---|---|---|
| | Aqueous alginate solution | | Aqueous calcium chloride solution | | | | | | | | | | |
| | DI | ALG | DI | CaCl₂ | DI | AM | MBA | APS | DI | AM | MBA | APS | ALG |
| Example 2 | 100 | 0.75 | 100 | 1.11 | 100 | 28.2 | 0.034 | 0.095 | 100 | 28.2 | 0.034 | 0.095 | 1.92 |
| Example 3 | 100 | 1.5 | 100 | 1.11 | 100 | 28.2 | 0.034 | 0.095 | 100 | 28.2 | 0.034 | 0.095 | 1.92 |
| Example 4 | 100 | 1.5 | 100 | 2.22 | 100 | 28.2 | 0.034 | 0.095 | 100 | 28.2 | 0.034 | 0.095 | 1.92 |
| Example 5 | 100 | 1.5 | 100 | 2.22 | 100 | 28.2 | 0.034 | 0.095 | 100 | 12 | 0.015 | 0.041 | 1.12 |
| Example 6 | 100 | 1.5 | 100 | 2.22 | 100 | 28.2 | 0.034 | 0.095 | 100 | 18 | 0.022 | 0.061 | 1.18 |
| Example 7 | 100 | 1.5 | 100 | 2.22 | 100 | 28.2 | 0.034 | 0.095 | 100 | 22 | 0.027 | 0.075 | 1.22 |

| | | | | | | | | | | | | | |
|---|---|---|---|---|---|---|---|---|---|---|---|---|---|
| * ALG: Alginate ** AM: Acrylamide | | | | | | | | | | | | | |

### Comparative Examples 1 to 3: Production of adipose tissue-like structure

An adipose tissue-like structure was produced in the same manner as in Examples 5 to 7, except that in the Examples 5 to 7, the soaking step of the network precursor solution in step (2) was not performed. The results are shown in FIG. 5.

### Experimental Example 1: Evaluation of adipose tissue-like structure

For adipose tissue-like structures according to Examples 1 to 7 and Comparative Examples 1 to 3, the appearance, sealing performance, elasticity, tactile sensation, and surface stickiness were measured, respectively.

In the case of the adipose tissue-like structures according to Examples 1 to 7, it was confirmed that the alginate in the form of multiple spherical particles contained in the network solution remains intact, and it exhibited a texture (soft) and elasticity similar to the fat layer tissue of the actual human body. In addition, when the adipose tissue-like structure produced in Examples was sutured using a sutured thread, it was found that the structure maintained its structure well without tearing or collapsing, and the outer surface of the structure was also confirmed to be non-sticky.

In the case of the adipose tissue-like structure according to Comparative Example 1, it was found that the network solution was not cured at all. In the case of Comparative Example 2, it was found that when external pressure is applied to the structure because the network solution is not fully cured, the alginate in the form of multiple spherical particles is detached, and the uncured network solution adheres to the surface of the structure, thereby causing stickiness. In the case of the adipose tissue-like structure according to Comparative Example 3, it can be seen that the curing process was not completed, thereby resulting in some stickiness on the surface.

Although the preferred Examples of the present invention have been described in detail above, the scope of the present invention is not limited thereto, and various modifications and improvements made by those skilled in the art using the basic concept of the present invention defined in the following claims also fall within the scope of the present invention.

## Claims

1. A composition for forming an adipose tissue-like structure, comprising:
a biocompatible polymer in a dispersed form in the composition;
a plurality of spherical particle-shaped biocompatible hydrogels;
an acrylamide-based monomer;
an initiator;
a crosslinking agent; and
a solvent.

2. The composition for forming an adipose tissue-like structure according to claim 1, wherein the biocompatible polymer and biocompatible hydrogel each comprise one or more of gelatin, gelatin methacrylate, collagen, alginate, fibrinogen, fibrin, hyaluronic acid, methyl cellulose, chitosan, chitin, Extracel^{™}, pectin, polylactic acid (PLA), polyacrylic acid (PAA), polyphosphoric acid, and synthetic peptide.

3. The composition for forming an adipose tissue-like structure according to claim 1, wherein the acrylamide-based monomer comprises one or more of acrylamide, N-isopropylacrylamide (NIPAM), N-tert-butyl acrylamide, N,N-diethylacrylamide, N-ethylacrylamide, N-n-propylacrylamide, N,N-ethylmethylacrylamide, N-isopropylmethacrylamide, N-hydroxyethylacrylamide, N-(isobutoxymethyl)acrylamide, N-tert-butyl methacrylamide, N,N-diethylmethacrylamide, and N-ethylmethacrylamide.

4. The composition for forming an adipose tissue-like structure according to claim 1, wherein the plurality of spherical particle-shaped biocompatible hydrogels comprise an ionic functional group.

5. The composition for forming an adipose tissue-like structure according to claim 4, wherein the ionic functional group is one or more selected from the group consisting of a carboxyl group, a hydroxyl group, a thiol group, an amine group, a sulfonate group, an ammonium group, a urea group, a thiourea group, an imidazole group, a phosphate group, a sulfonic acid group, and a phosphonic acid group.

6. The composition for forming an adipose tissue-like structure according to claim 1, wherein the biocompatible polymer in a dispersed form in the composition is contained in an amount of 0.2 to 1 parts by weight based on the total 100 parts by weight of the composition.

7. The composition for forming an adipose tissue-like structure according to claim 1, wherein the plurality of spherical particle-shaped biocompatible hydrogels are contained in an amount of 40 to 60 parts by weight based on the total 100 parts by weight of the composition.

8. The composition for forming an adipose tissue-like structure according to claim 1, wherein the acrylamide-based monomer is contained in an amount of 3 to 16 parts by weight based on the total 100 parts by weight of the composition.

9. The composition for forming an adipose tissue-like structure according to claim 1, wherein the plurality of spherical particles are in the form of granules.

10. The composition for forming an adipose tissue-like structure according to claim 1, wherein in the plurality of spherical particle-shaped biocompatible hydrogels, the average particle size of each individual particle is 0.1 to 3 mm.

11. The composition for forming an adipose tissue-like structure according to claim 1, wherein the composition is formed by soaking the plurality of spherical particle-shaped biocompatible hydrogels in a solution containing the biocompatible polymer in a dispersed form in the composition, an acrylamide-based monomer, an initiator, and a crosslinking agent in a solvent.

12. A method for producing an adipose tissue-like structure, comprising the steps of:
(1) preparing a plurality of spherical particle-shaped biocompatible hydrogels by dropping a biocompatible hydrogel into a solution containing a polyvalent metal ion compound;
(2) preparing a mixed solution by adding the plurality of spherical particle-shaped biocompatible hydrogels prepared in step (1) into a network solution containing an acrylamide-based monomer, an initiator, a crosslinking agent, a biocompatible polymer, and a solvent; and
(3) curing the mixed solution of the step (2).

13. The method for producing an adipose tissue-like structure according to claim 12, wherein the mixing ratio of the plurality of spherical particle-shaped biocompatible hydrogels from the step (1) and the network solution from the step (2) is a weight ratio of 1:0.7 to 1:1.3.

14. The method for producing an adipose tissue-like structure according to claim 12, further comprising the step of soaking the plurality of spherical particle-shaped biocompatible hydrogels in a network precursor solution containing an acrylamide-based monomer, an initiator, and a crosslinking agent, after the step (1).

15. The method for producing an adipose tissue-like structure according to claim 12, wherein the polyvalent metal ion compound may comprise one or more of sodium chloride, zirconium chloride, iron chloride, calcium chloride, magnesium chloride, aluminum chloride, aluminum sulfate, potassium alum, iron chloride alum, ammonium alum, ferric sulfate, aluminum hydroxide, aluminum silicate, aluminum phosphate, ferric citrate, magnesium oxide, calcium phosphate, calcium carbonate, calcium oxide, zinc oxide, and zinc sulfate.
